(19) Europäisches Patentamt
European Patent Office
Office européen des brevets

(11) Veröffentlichungsnummer: 0 223 142 A1

# (12) EUROPÄISCHE PATENTANMELDUNG

(21) Anmeldenummer: 86115196.7

(51) Int. Cl.⁴: C07D 249/08 , A01N 43/653

(22) Anmeldetag: 03.11.86

(30) Priorität: 15.11.85 DE 3540527

(43) Veröffentlichungstag der Anmeldung: 27.05.87 Patentblatt 87/22

(84) Benannte Vertragsstaaten: BE CH DE FR GB IT LI NL

(71) Anmelder: BAYER AG
Konzernverwaltung RP Patentabteilung
D-5090 Leverkusen 1 Bayerwerk(DE)

(72) Erfinder: Kraatz, Udo, Dr.
Andreasstrasse 22a
D-5090 Leverkusen(DE)
Erfinder: Regel, Erik, Dipl. Ing.
Untere Bergerheide 26
D-5600 Wuppertal 1(DE)
Erfinder: Lürssen, Klaus, Dr.
August-Kierspel-Strasse 151
D-5060 Bergisch-Gladbach 2(DE)

# (54) (-)-Antipode des (E)-1-(4-Chlorphenyl)-5-fluor-4,4-dimethyl-3-hydroxy-2-(1,2,4-triazol-l-yl)-pent-l-ens.

(57) Neuer (-)-Antipode des (E)-1-(4-Chlorphenyl)-5-fluor-4,4-dimethyl-3-hydroxy-2-(1,2,4-triazol-1-yl)-pent-1-ens der Formel

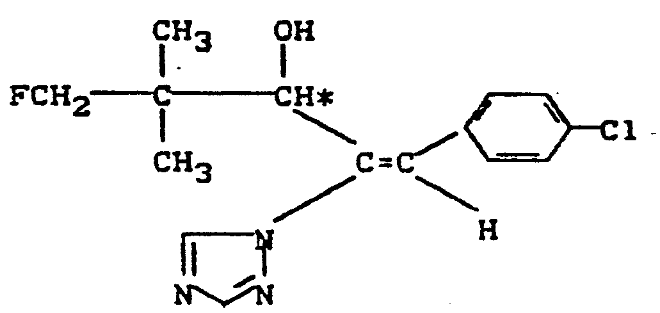

(I)

mehrere Verfahren zur Herstellung des neuen Wirkstoffes und dessen Verwendung zur Regulierung des Pflanzenwachstums.

EP 0 223 142 A1

## (-)-Antipode des (E)-1-(4-Chlorphenyl)-5-fluor-4,4-dimethyl-3-hydroxy-2-(1,2,4-triazol-1-yl)-pent-1-ens

Die vorliegende Erfindung betrifft den neuen (-)-Antipoden*⁾ des (E)-1-(4-Chlorphenyl)-5-fluor-4-dimethyl-3-hydroxy-2-(1,2,4-triazol-1-yl)-pent-1-ens, mehrere Verfahren zu dessen Herstellung und dessen Verwendung zur Regulierung des Pflanzenwachstums.

Es ist bereits bekannt, daß das Racemat des (E)-1-(4-Chlorphenyl)-5-fluor-4,4-dimethyl-3-hydroxy-2-(1,2,4-triazol-1-yl)-pent-1-ens pflanzenwuchsregulierende Eigenschaften besitzt (vgl. EP-OS 0 044 425). Die Wirksamkeit dieses Produktes ist gut; jedoch ist der bei sehr niedrigen Aufwandmengen erzielte Effekt nicht immer befriedigend.

*⁾ Unter dem (-)-Antipoden ist hier jeweils dasjenige Enantiomere zu verstehen, das die Schwingungsebene von linear polarisiertem Licht der Natrium-D-Linie nach links dreht.

Es wurde nun der (-)-Antipode des (E)-1-(4-Chlorphenyl)-5-fluor-4,4-dimethyl-3-hydroxy-2-(1,2,4-triazol-1-yl)-pent-1-ens der Formel

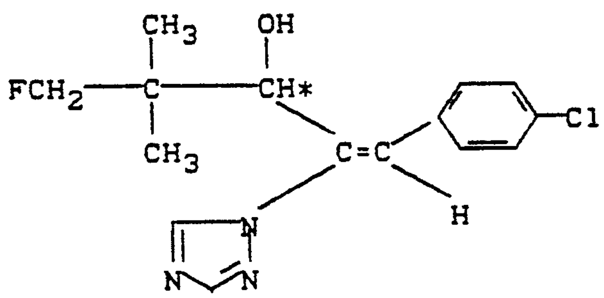

(I)

gefunden.

Weiterhin wurde gefunden, daß man den neuen (-)-Antipoden des (E)-1-(4-Chlorphenyl)-5-fluor-4,4-dimethyl-3-hydroxy2-(1,2,4-triazol-1-yl)-pent-1-ens der Formel (I) erhält, wenn man

a) in einer ersten Stufe racemisches (E)-1-(4-Chlorphenyl)-5-fluor-4,4-dimethyl-3-hydroxy-2-(1,2,4-triazol-1-yl)-pent-1-en der Formel

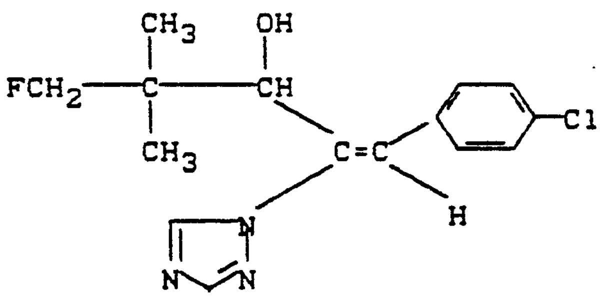

(Ia)

mit einem optisch aktiven Säurehalogenid der Formel

R -X -Hal (II)

in welcher

R für einen optisch aktiven Rest steht,

X für -CO-, $-SO_2$-oder $-O-CH_2-CO$-steht und

Hal für Halogen steht,

in Gegenwart eines Verdünnungsmittels und gegebenenfalls in Gegenwart von Basen umsetzt, dann die so erhaltenen diastereomeren Ester der Formel

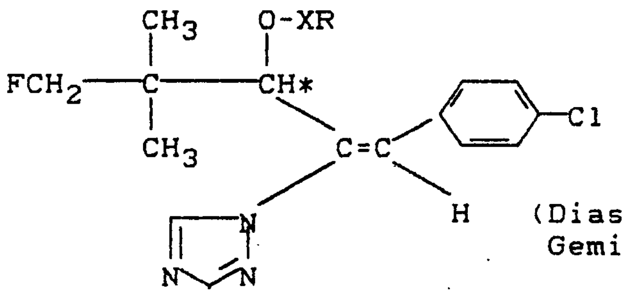

(III)
(Diastereomeren-Gemisch)

in welcher
R und X die oben angegebene Bedeutung haben, aufgrund ihrer unterschiedlichen physikalischen Eigenschaften trennt
und danach in einer zweiten Stufe den (-)-Antipoden des (E)-1-(4-Chlorphenyl)-5-fluor-4,4-dimethyl-3-hydroxy-2-(1,2,4-triazol-1-yl)-pent-1-ens aus dem entsprechenden Ester mit Hilfe von Basen in Gegenwart eines Verdünnungsmittels in Freiheit setzt,
oder

b) (E)-1-(4-Chlorphenyl)-5-fluor-4,4-dimethyl-2-(1,2,4-triazol-1-yl)-pent-1-en-3-on der Formel

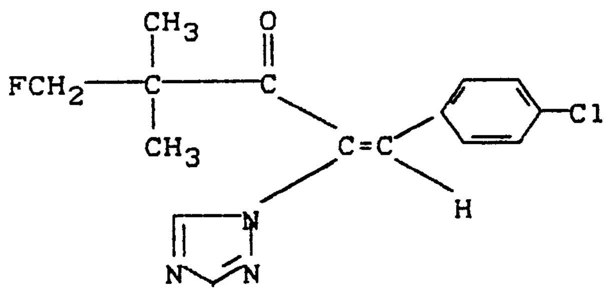

(IV)

mit einem komplexen Hydrid in Gegenwart eines inerten organischen Verdünnungsmittels sowie in Gegenwart eines chiralen Aminoalkohols und gegebenenfalls in Gegenwart eines Amins umsetzt.

Schließlich wurde gefunden, daß sich der neue (-)-Antipode des (E)-1-(4-Chlorphenyl)-5-fluor-4,4-dimethyl-3-hydroxy-2-(1,2,4-triazol-1-yl)-pent-1-ens der Formel (I) durch eine hervorragende pflanzenwuchsregulierende Wirksamkeit auszeichnet.

Überraschenderweise besitzt der neue (-)-Antipode des (E)-1-(4-Chlorphenyl)-5-fluor-4,4-dimethyl-3-hydroxy-2-(1,2,4-triazol-1-yl)-pent-1-ens der Formel (I) wesentlich bessere pflanzenwuchsregulierende Eigenschaften als das entsprechende Racemat, das aus dem Stand der Technik als hoch wirksamer Pflanzenwuchsregulator bekannt ist. Im übrigen konnte nicht erwartet werden, daß der erfindungsgemäße Wirkstoff sich durch sehr gute pflanzenwuchsregulierende Wirksamkeit hervorhebt, während der (+)-Antipode des (E)-1-(4-Chlorphenyl)-5-fluor-4,4-dimethyl-3-hydroxy-2-(1,2,4-triazol-1-yl)-pent-1-ens als Pflanzenwachstumsregulator weitgehend inaktiv ist.

Die erfindungsgemäße Verbindung ist durch die Formel (I) definiert. In dieser Formel ist das asymmetrisch substituierte Kohlenstoffatom durch ein (*) gekennzeichnet.

Verwendet man racemisches (E)-1-(4-Chlorphenyl-5-fluor4,4-dimethyl-3-hydroxy-2-(1,2,4-triazol-1-yl)-pent-1-en als Ausgangsstoff, (-)-Menth-3-yloxy-acetylchlorid als optisch aktives Säurehalogenid, Triethylamin und 4-Dimethylaminopyridin (DMAP) als Hilfsbasen zur Veresterung (1. Stufe) und ein Gemisch aus Natriumhydroxid, Wasser und Methanol zur Esterverseifung (2. Stufe), so kann der Verlauf des erfindungsgemäßen Verfahrens (a) durch das folgende Formelschema wiedergegeben werden:

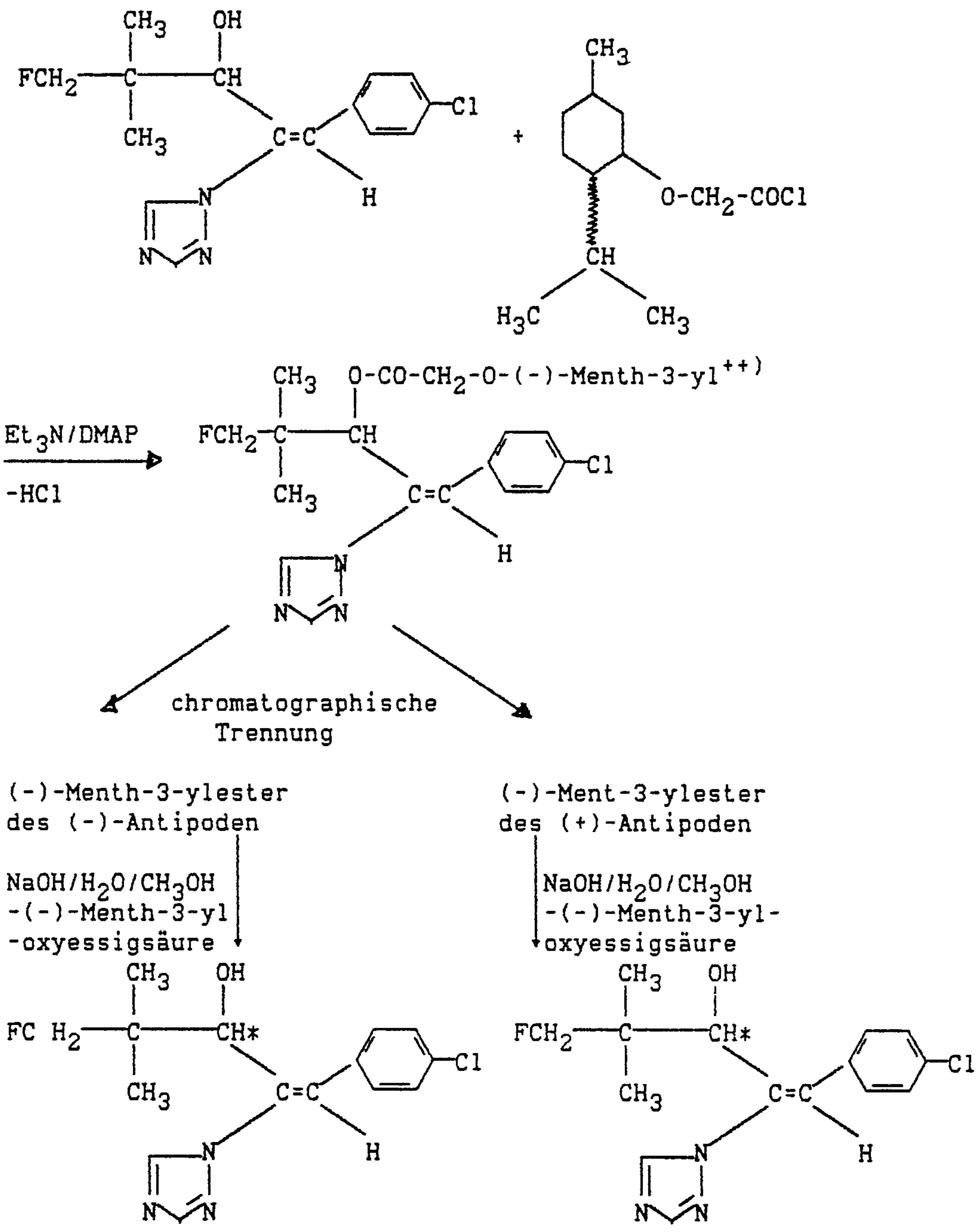


(-)-Antipode (+)-Antipode

**)
(-)-Menth-3-yl = 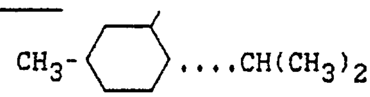

Verwendet man (E)-1-(4-Chlorphenyl)-5-fluor-4,4-dimethyl-2-(1,2,4-triazol-1-yl)-pent-1-en-3-on als Ausgangsstoff, Lithiumaluminiumhydrid als Reduktionsmittel, (-)-Antipoden des N-Methylephedrins als chirales Hilfsreagenz und N-Ethyl-anilin als zusätzliches Amin, so kann der Verlauf des erfindungsgemäßen Verfahrens (b) durch das folgende Formelschema wiedergegeben werden:

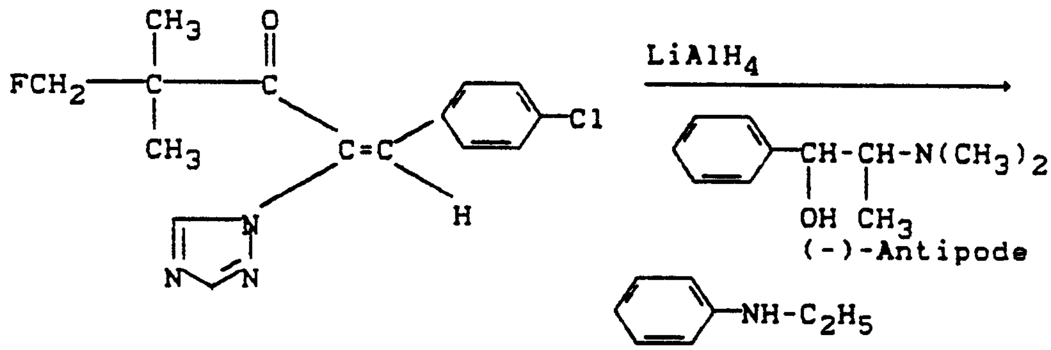


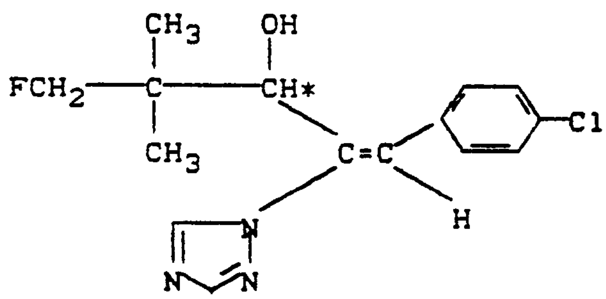

(-)-Antipode

Das bei dem erfindungsgemäßen Verfahren (a) als Ausgangsstoff benötigte Racemat des (E)-1-(4-Chlorphenyl)-5-fluor-4,4-dimethyl-3-hydroxy-2-(1,2,4-triazol-1-yl)-pent-1-ens der Formel (Ia) ist bekannt (vgl. EP-OS 0 044 425).

Die bei dem erfindungsgemäßen Verfahren (a) weiterhin als Ausgangssubstanzen benötigten optisch aktiven Säurehalo genide sind durch die Formel (II) allgemein definiert. In dieser Formel steht R vorzugsweise für (+)-Bromcampher-8-yl, (+)-Campher-10-yl oder (-)-Menth-3-yl.

X steht für die Reste -CO-, $-SO_2-$und $-O-CH_2-CO-$,

und Hal steht vorzugsweise für Chlor oder Brom.

Als Beispiele für Verbindungen der Formel (II) seien genannt:

(+)-3-Brom-campher-8-sulfonsäurechlorid,
(+)-Campher-10-sulfonsäurechlorid
(+)-3-Brom-campher-8-sulfonsäurebromid
(+)-Campher-10-sulfonsäurebromid
(-)-Menth-3-yl-oxyacetylchlorid
(-)-Menth-3-yl-oxyacetylbromid.

Die optisch aktiven Säurehalogenide sind bekannt oder lassen sich nach bekannten Methoden in einfacher Weise herstellen.

Als Verdünnungsmittel kommen für die Durchführung der 1. Stufe (Veresterung) des erfindungsgemäßen Verfahrens (a) alle inerten organischen Lösungsmittel in Frage. Hierzu gehören vorzugsweise Kohlenwasserstoffe, wie Benzin, Benzol, Toluol oder Xylol, halogenierte Kohlenwasserstoffe, wie Dichlormethan, Chloroform, Tetrachlorkohlenstoff, Chlorbenzol oder Dichlorbenzol, Ether wie Diethylether, Tetrahydrofuran oder Dioxan, Nitrile, wie Acetonitril oder Propionitril oder Ester, wie Essigsäureethylester.

Die erste Stufe des erfindungsgemäßen Verfahrens (a) wird vorzugsweise in Gegenwart von Basen durchgeführt. Hierbei können alle üblichen organischen oder anorganischen Basen eingesetzt werden. Vorzugsweise verwendbar sind Alkalihydroxide oder Alkalicarbonate, wie beispielsweise Natriumhydroxid, Natriumcarbonat oder Natriumhydrogencarbonat, ferner niedere tertiäre Alkylamine, Cycloalkylamine, Arylalkylamine oder Arylamine, wie beispielsweise Triethylamin, N,N-Dimethylbenzylamin, Pyridin, 1,4-Diazabicyclo-[2,2,2]-octan oder 1,5-Diazabicyclo-[4,3,0]-non-5-en. Besonders bevorzugt verwendet man ein Gemisch aus Triethylamin und dem hochnucleophilen 4-Dimethylaminopyridin.

Die Reaktiionstemperaturen können bei der Durchführung der 1. Stufe des erfindungsgemäßen Verfahrens (a) in einem größeren Bereich variiert werden. Im allgemeinen arbeitet man zwischen -20° C und +120° C, vorzugsweise zwischen 0° C und 60° C.

Bei der Durchführung der 1. Stufe des erfindungsgemäßen Verfahrens (a) setzt man auf 1 Mol der racemischen Ausgangsverbindung der Formel (Ia) vorzugsweise 1 bis 1,5 Mol optisch aktives Säurehalogenid der Formel (II) und 2 bis 3 Mol Base ein. Die Isolierung des Diastereomeren-Gemisches erfolgt nach üblichen Methoden. Im allgemeinen geht man so vor, daß man nach beendeter Umsetzung mit Wasser versetzt, das entstehende Gemisch mehrfach mit einem mit Wasser wenig mischbaren organischen Lösungsmittel extrahiert, die vereinigten organischen Phasen trocknet und unter vermindertem Druck einengt.

Die Trennung der diastereomeren Ester der Formel (III) kann nach den für derartige Zwecke geeigneten Methoden vorgenommen werden, also zum Beispiel durch fraktionierte Kristallisation oder auch mit Hilfe von chromatographischen Verfahren.

Besonders bevorzugt wendet man säulenchromatographische Trennverfahren an, wie z.B. die Hochdruckfiltration über eine Kieselsäule mit einem Elutionsgemisch aus Hexan, Tetrachlorkohlenstoff und Propionitril.

Als Verdünnungsmittel für die 2. Stufe (Esterverseifung) des erfindungsgemäßen Verfahrens (a) kommen ebenfalls inerte organische Lösungsmittel in Frage. Besonders bevorzugt verwendet man Alkohole, wie beispielsweise Methanol, Ethanol oder Propanol.

Die Freisetzung des erfindungsgemäßen Wirkstoffes erfolgt in der zweiten Stufe des erfindungsgemäßen Verfahrens (a) mit Hilfe von Basen. Bevorzugt verwendet man dabei starke wäßrige anorganische Basen, wie Natriumhydroxid oder Kaliumhydroxid in Wasser.

Die Reaktionstemperaturen können bei der Durchführung der 2. Stufe des erfindungsgemäßen Verfahrens (a) ebenfalls in einem größeren Bereich variiert werden. Im allgemeinen arbeitet man zwischen 0° C und 80° C, vorzugsweise zwischen 10° C und 60° C.

Bei der Durchführung der 2. Stufe des erfindungsgemäßen Verfahrens (a) setzt man auf 1 Mol des jeweiligen diastereomeren Esters der Formel (III) im allgemeinen 1 bis 3 Mol, vorzugsweise 1 bis 2 Mol Base ein.

Die Isolierung des erfindungsgemäßen Wirkstoffes erfolgt bei dem erfindungsgemäßen Verfahren (a) nach üblichen Methoden. Im allgemeinen verfährt man in der Weise, daß man das Reaktionsgemisch mit Wasser versetzt, dann mehrfach mit einem mit Wasser wenig mischbaren organischen Lösungsmittel extrahiert, die vereinigten organischen Phasen trocknet und durch Abziehen des Lösungsmittels unter vermindertem Druck einengt und den verbleibenden Rückstand gegebenenfalls durch Umkristallisation oder durch Waschen mit einem organischen Lösungsmittel von eventuell vorhandenen Verunreinigungen befreit.

Bei der Durchführung der zweiten Stufe des erfindungsgemäßen Verfahrens (a) wird jeweils derjenige diastereomere Ester der Formel (III) eingesetzt, aus dem durch Behandlung mit Base der erfindungsgemäße (-)-Antipode des (E)-1-(4-Chlorphenyl)-5-fluor-4,4-dimethyl-3-hydroxy-2-(1,2,4-triazol-1-yl)-pent-1-ens in Freiheit gesetzt wird.

Nach dem erfindungsgemäßen Verfahren (a) läßt sich auch der (+)-Antipode des (E)-1-(4-Chlorphenyl)-5-fluor-4,4-dimethyl-3-hydroxy-2-(1,2,4-triazol-1-yl)-pent-1-ens darstellen. Man geht dabei so vor, daß man nach der Trennung der diastereomeren Ester jeweils diejenige Verbindung der Formel (III), die den (+)-Antipoden enthält, mit Base in Gegenwart eines Verdünnungsmittels behandelt. Die Reaktionsbedingungen entsprechen denjenigen, die im Falle des erfindungsgemäßen Verfahrens (a) bei der Durchführung der zweiten Stufe in Frage kommen.

Das bei der Durchführung des erfindungsgemäßen Verfahrens (b) als Ausgangsmaterial benötigte (E)-1-(4-Chlorphenyl)-5-fluor-4,4-dimethyl-2-(1,2,4-triazol-1-yl)-pent-1-en-3-on der Formel (IV) ist bereits bekannt - (vgl. DE-OS 30 25 242).

Bei der Durchführung des erfindungsgemäßen Verfahrens (b) fungieren komplexe Hydride als Reduktionsmittel. Vorzugsweise verwendbar ist Lithiumaluminiumhydrid.

Als Verdünnungsmittel können bei der erfindungsgemäßen Umsetzung (b) alle für derartige Reaktionen üblichen inerten organischen Solventien eingesetzt werden. Vorzugsweise in Betracht kommen Ether, wie Diethylether, Tetrahydrofuran und tert.-Butyl-methyl-ether.

Als chirale Hilfsreagentien kommen bei der Durchführung des erfindungsgemäßen Verfahrens (b) optisch aktive Aminoalkohole in Frage. Besonders bevorzugt ist der (-)-Antipode des N-Methyl-ephedrins.

Als Amine, die bei der Durchführung des erfindungsgemäßen Verfahren (b) gegebenenfalls eingesetzt werden können, kommen vorzugsweise sekundäre Amine in Betracht. Besonders bevorzugt ist N-Ethylanilin.

Die Reaktionstemperaturen können bei der Durchführung des erfindungsgemäßen Verfahrens (b) innerhalb eines bestimmten Bereiches variiert werden. Im allgemeinen arbeitet man bei Temperaturen zwischen -70° C und +50° C, vorzugsweise zwischen -20° C und +40° C.

Das erfindungsgemäße Verfahren (b) wird im allgemeinen unter Normaldruck durchgeführt.

Vorzugsweise arbeitet man unter Schutzgasatmosphäre. Als Schutzgase können dabei alle üblichen unter den Reaktionsbedingungen inerten Gase eingesetzt werden. Vorzugsweise verwendbar sind Stickstoff und Argon.

Bei der Durchführung des erfindungsgemäßen Verfahrens (b) setzt man auf 1 Mol an (E)-1-(4-Chlorphenyl)-5-fluor-4,4-dimethyl-2-(1,2,4-triazol-1-yl)-pent-1-en-3-on der Formel (IV) im allgemeinen 1 bis 3 Mol, vorzugsweise 1,3 bis 2 Mol, eines komplexen Hydrids und 1 bis 3 Mol, vorzugsweise 1,3 bis 2 Mol, an chiralem Aminoalkohol sowie gegebenenfalls 2 bis 4 Mol, vorzugsweise 2,5 bis 3,5 Mol, an zusätzlichem Amin ein.

Im allgemeinen geht man bei der Durchführung des erfindungsgemäßen Verfahrens (b) so vor, daß man chiralen Aminoalkohol, gegebenenfalls in einem organischen Verdünnungsmittel gelöst, bei Temperaturen zwischen 0° C und 50° C, vorzugsweise zwischen 10° C und 40° C, in eine Suspension eines komplexen Hydrids in einem organischen Verdünnungsmittel eintropft, anschließend gegebenenfalls Amin zugibt und danach bei Temperaturen zwischen -70° C und 0° C, vorzugsweise zwischen -70° C und -40° C, eine Lösung von (E)-1-(4-Chlorphenyl)-5-fluor-4,4-dimethyl-2-(1,2,4-triazol-1-yl)-pent-1-en-3-on der Formel (IV) in einem organischen Verdünnungsmittel zutropft. Die Aufarbeitung erfolgt nach üblichen Methoden. Im allgemeinen verfährt man in der Weise, daß man Wasser hinzufügt, das Reaktionsgemisch ansäuert, die organische Phase abtrennt, die wäßrige Phase mehrfach mit einem mit Wasser wenig mischbaren organischen Solvens extrahiert, die vereinigten organischen Phasen mit Wasser wäscht und danach einengt. Der verbleibende Rückstand kann durch Digerieren mit geeigneten organischen Solventien oder durch Umkristallisation weiter gereinigt werden.

Der erfindungsgemäße Wirkstoff greift in den Metabolismus der Pflanzen ein und kann deshalb als Wachstumsregulator eingesetzt werden.

Für die Wirkungsweise von Pflanzenwachtumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachtumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergärten, Park-und Sportanlagen, an Straßenrändern, auf Flughäfen oder in Obstanlagen reduziert werden. Von Bedeutung ist auch die Hemmung des Wuchses von krautartigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten-und Fruchtbildung zugute kommen als den vegetativen Pflanzenteilen.

Mit Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch, daß mehr Assimilate gebildet werden, so daß mehr oder größere Früchte enstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerden läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen-oder Knospenruhe der Pflanzen beeinflußt werden, so daß die Pflanzen, wie z.B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Der erfindungsgemäße Wirkstoff kann in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen des Wirkstoffes mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diateomeenerde und synthetische Gesteinsmehle, wie hochdisperse Kieselsäure, Aluminiumoxid und Silikate. Als feste Trägerstoffe für Granulate kommen in Frage: z.B.

gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier-und/oder -schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo-und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Der erfindungsgemäße Wirkstoff kann in den Formulierungen in Mischung mit anderen bekannten Wirkstoffen vorliegen, wie Fungizide, Insektizide, Akarizide und Herbizide, sowie in Mischungen mit Düngemitteln und anderen Wachstumsregulatoren.

Der Wirkstoff kann als solcher, in Form seiner Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, den Wirkstoff nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung des Wachstumsregulators in einem bevorzugten Zeitraum vorgenommen wird, dessen genaue Abgrenzung sich nach den klimatischen und vegetativen Gegebenheiten richtet.

Die Herstellung und die Verwendung des erfindungsgemäßen Wirkstoffs werden durch die folgenden Beispiele veranschaulicht.

Herstellungsbeispiele

Beispiel 1

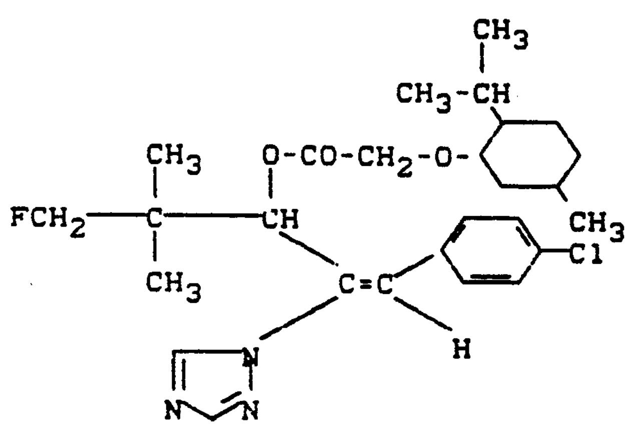

(III-1)

1. Stufe

Eine Lösung von 14,7 g (0,0475 Mol) racemischem (E)-1-(4-Chlorphenyl)-5-fluor-4,4-dimethyl-3-hydroxy-2-(1,2,4-triazol-1-yl)-pent-1-en, 5,8 g (0,0475 Mol) 4-Dimethylamino-pyridin und 2 g Triethylamin in 70 ml absolutem Tetrahydrofuran wird unter Rühren und Eiskühlung tropfenweise mit 11 g (0,0475 Mol) (-)-Menth-3-yloxy-acetylchlorid versetzt. Nach beendeter Zugabe wird zunächst 3 Stunden bei 20° C nachgerührt und dann 16 Stunden bei Raumtemperatur stehengelassen. Zur Aufarbeitung gießt man das Reaktionsgemisch in Wasser, extrahiert mehrfach mit Methylenchlorid, wäscht die organische Phase mit verdünnter wäßriger Salzsäure, trocknet über Magnesiumsulfat und engt unter vermindertem Druck ein. Man erhält 21,5 g (89.7 % der Theorie) an (E)-1-(4-Chlorphenyl)-5-fluor-4,4-dimethyl-3-(-)-(menth-3-yloxy-acetyloxy)-2-(1,2,4-triazol-1-yl)-pent-1-en Diastereomeren-Gemisch als hochviskoses Öl.

Die Trennung in die diastereomeren Einzelkomponenten erfolgt mittels hochauflösender Flüssigkeits - Chromatographie (= High performance liquid chromatography = HPLC) an einer Kieselgelsäule (Merck Si 60; Korngröße 5-20 µ) mit n-Hexan/tert.-Butyl-methylether = 5:2 als Elutionsmittelgemisch.

Als erste Fraktion erhält man eine Lösung, von der nach dem Einengen 6,0 g (55,8 % der Theorie) an (-)-Menth-3-yloxy-acetylester des (+)-Enantiomeren des (E)-1-(4-Chlorphenyl)-5-fluor-4,4-dimethyl-3-hydroxy-2-(1,2,4-triazol-1-yl)-pent-1-ens vom Brechungsindex $n_D^{20}$ = 1,5250 isoliert werden.

Als zweite Fraktion eluiert man eine Lösung, die nach dem Einengen 4,3 g (40% der Theorie) an (-)-Menth-3-yloxy-acetylester des (-)-Enantiomeren des (E)-1-(4-Chlorphenyl)-5-fluor-4,4-dimethyl-3-hydroxy-2-(1,2,4-triazol-1-yl)-pent-1-ens vom Brechungsindex $n_D^{20}$ = 1,5232 liefert. $C_{27}H_{36}ClFN_3O_3$ (504.5)

2. Stufe

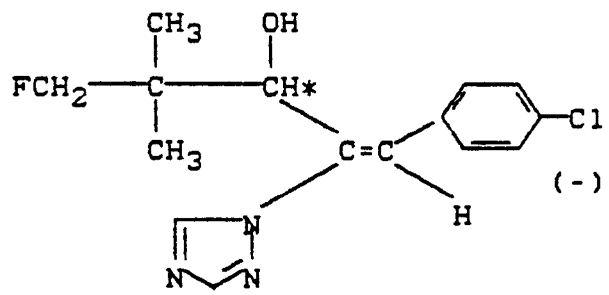

(-)-Antipode

4,0g (7,9 mMol) der 2. Esterfraktion werden in 80 ml Methanol mit einer Lösung von 1 g (0,025 Mol) Natriumhydroxid in 6 ml Wasser bei 20° C versetzt und 16 Stunden bei Raumtemperatur gerührt. Zur Aufarbeitung verdünnt man mit 150 ml Wasser, extrahiert dreimal mit jeweils 120 ml Dichlormethan, wäscht die vereinigten organischen Extrakte mit Wasser und trocknet über Magnesiumsulfat. Nach Entfernen des Lösungsmittels wird der feste Rückstand mit Cyclohexan verrührt und abgesaugt. Man erhält 2,0 g (82 % der Theorie) des (-)-Antipoden des (E)-2-(4-Chlorphenyl)-5-fluor-4,4-dimethyl-3-hydroxy-2-(1,2,4-triazol-1-yl)-pent-1-ens vom Schmelzpunkt 153° C.

$[\alpha]_D^{20}$ : -12,8° (C=0,484/$CHCl_3$)

Die optische Reinheit beträgt 88% e.e.

Herstellung des Ausgangsproduktes der Formel

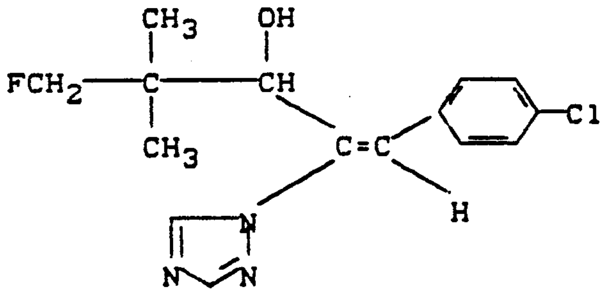

(Racemat)

45,7 g (0,154 Mol) (E)-1-(4-Chlorphenyl)-5-fluor-4,4-dimethyl-2-(1,2,4-triazol-1-yl)-pent-1-en-3-on und 11,5 g (0,103 Mol) Calciumchlorid werden in 350 ml Isopropanol suspendiert und bei -5° C mit einer Lösung von 4,1 g (0,108 Mol) Natriumborhydrid in 50 ml Wasser tropfenweise versetzt. Nach 4 Stunden wird das Reaktionsgemisch auf Raumtemperatur erwärmt und 15 Stunden bei Raumtemperatur nachgerührt. Nach Entfernen des Lösungsmittels wird der ölige Rückstand in essigsaures Wasser eingerührt und mit Methylenchlorid extrahiert. Die vereinigten Methylenchlorid-Extrakte werden mit Wasser gewaschen, über Natriumsulfat getrocknet und unter vermindertem Druck eingeengt. Der ölige Rückstand kristallisiert beim Verrühren mit Diethylether.

Man erhält 38,7 g (81,4% der Theorie) an (E)-1-(4-Chlorphenyl)-5-fluor-4,4-dimethyl-3-hydroxy-2-(1,2,4-triazol-1-yl)-pent-1-en in Form einer Festsubstanz vom Schmelzpunkt 148° C.

Beispiel 2

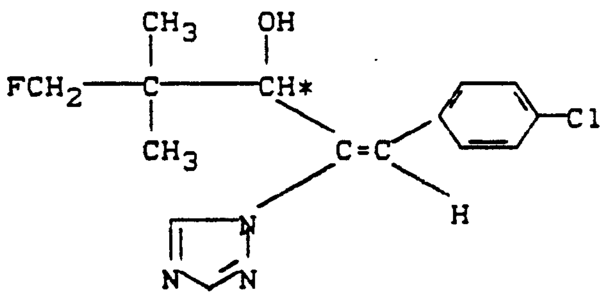

(-)-Antipode

Zu einer Suspension von 2,7 g (70 mMol) Lithiumaluminiumhydrid in 70 ml absolutem Diethylether wird bei 20° C unter Rühren eine Lösung von 12,5 g (70 mMol) (-)-N-Methylephedrin in 140 ml absolutem Diethylether getropft. Danach wird das Reaktionsgemisch 30 Minuten unter Rückfluß erhitzt. Nach dem Abkühlen auf 20° C tropft man 17 g (140 mMol) N-Ethylanilin hinzu und läßt dann 1 Stunde unter Rückfluß sieden. Anschließend kühlt man das Reaktionsgemisch auf -70° C ab und tropft bei dieser Temperatur eine Lösung von 6,2 g (20 mMol) (E)-1-(4-Chlorphenyl)-5-fluor4,4-dimethyl-2-(1,2,4-triazol-1-yl)-pent-1-en-3-on in 120 ml Diethylether dazu. Nach fünfstündigem Rühren bei -70° C läßt man die Temperatur des Reaktionsgemisches allmählich auf 20° C ansteigen, läßt 16 Stunden bei Raumtemperatur stehen und hydrolysiert dann durch langsame Zugabe von Wasser. Anschließend wird mit verdünnter wäßriger Salzsäure angesäuert. Die organische Phase wird abgetrennt, zweimal mit verdünnter wäßriger Salzsäure und einmal mit Wasser gewaschen und danach unter vermindertem Druck eingeengt. Der verbleibende Rückstand wird mit Cyclohexan verrührt und abgesaugt. Man erhält 3,5 g (56% der Theorie) an (-)-Antipoden des (E)-1-(4-Chlorphenyl)-5-fluor-4,4-dimethyl-3-hydroxy-2-(1,2,4-triazol-1-yl)-pent-1-ens vom Schmelzpunkt 136° C.

$[\alpha]_D^{20}$ = -7,3° (c = 0,630/$CHCl_3$)

Die optische Reinheit beträgt 72% e.e.

Herstellung des Ausgangsproduktes der Formel

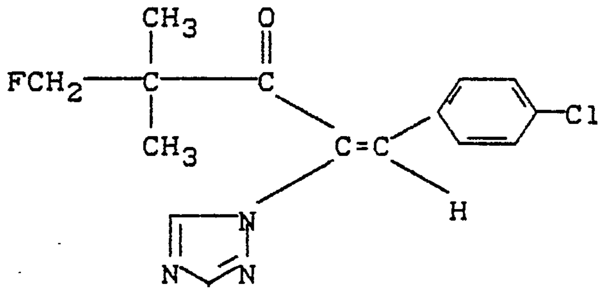

(IV)

185 g (1 Mol) 1-Fluor-2,2-dimethyl-4-(1,2,4-triazol-1-yl)-butan-3-on, 140,5 g (1 Mol) 4-Chlorbenzaldehyd sowie 10 ml Piperidin und 30 ml Eisessig werden in 400 ml Toluol 15 Stunden unter Rückfluß erhitzt, wobei das entstandene Reaktionswasser azeotrop entfernt wird. Danach wird das Reaktionsgemisch mit Wasser neutral gewaschen, über Natriumsulfonat getrocknet und unter vermindertem Druck eingeengt. Man erhält 279.4 g (90,9% der Theorie) rohes 1-(4-Chlorphenyl)-5-fluor-4,4-dimethyl-4-(1,2,4-triazol-1-yl)-pent-1-en-3-on vom Brechungsindex $n_D^{20}$ :1,5824 als E/Z-Isomerengemisch.

279,4 g (0,91 Mol) 1-(4-Chlorphenyl)-5-fluor-4,4-dimethyl-4-(1,2,4-triazol-1-yl)-pent-1-en-3-on werden in 3,5 l Dioxan gelöst und 22 Stunden bei Raumtemperatur durch Bestrahlung mit zwei Quecksilberhochdruckstrahlen TQ 150 isomerisiert. Nach Entfernen des Lösungsmittels wird der verbleibende ölige Rückstand durch Verrühren mit Diethylether zur Kristallisation gebracht. Man erhält 181,6 g (65% der Theorie) reines E-Isomeres vom Schmelzpunkt 109° C.

Beispiel 3

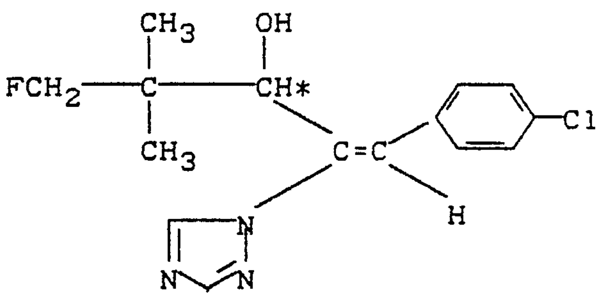

(+)-Antipode

5,5 g (10,8 mMol) desjenigen Diastereomeren, das aus der 1. Fraktion der im Beispiel 1 (erste Stufe) beschriebenen Eluierung gewonnen wurde, werden in 80 ml Methanol mit einer Lösung von 1 g (25 mMol) Natriumhydroxid in 6 ml Wasser versetzt und 16 Stunden bei 20° C gerührt. Zur Aufarbeitung verdünnt man mit 150 ml Wasser, extrahiert dreimal mit jeweils 120 ml Dichlormethan, wäscht die vereinigten organischen Extrakte mit Wasser und trocknet über Magnesiumsulfat. Im Vakuum wird das Lösungsmittel entfernt, der feste Rückstand wird mit Cyclohexan verrührt und abgesaugt.

Man erhält 3,2 g (93 % der Theorie) des (+)-Antipoden des (E)-1-(4-Chlorphenyl)-5-fluor-4,4-dimethyl-3-hydroxy-2-(1,2,4-triazol-1-yl)-pent-1-ens vom Schmelzpunkt 154° C.

$[\alpha]_D^{20}$ : + 10,1° (C = 0.615/$CHCl_3$)

Die optische Reinheit beträgt 93% e.e.

In den folgenden Verwendungsbeispielen wurden die nachstehend angegebenen Verbindungen bezüglich ihrer pflanzenwuchsregulierenden Eigenschaften geprüft:

(A) = 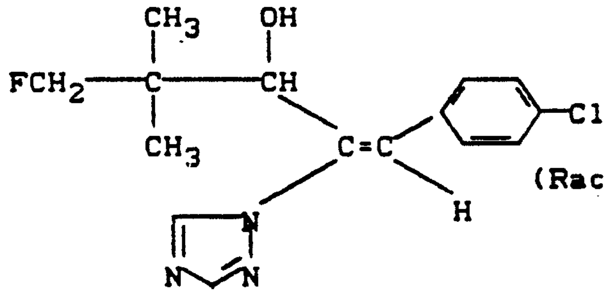 (Racemat)

(EP-OS 0 044 425)

(B) = 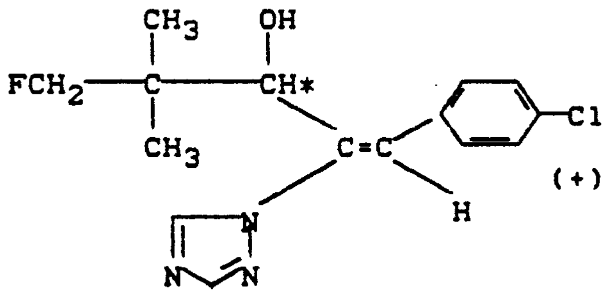 (+)-Antipode

(I) = 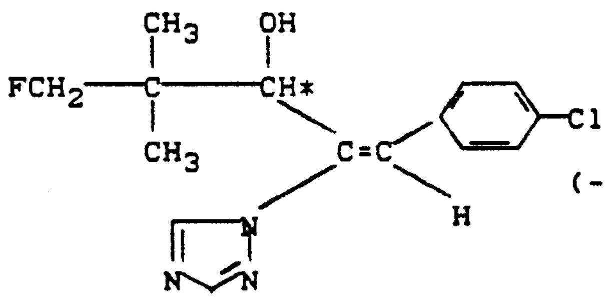 (-)-Antipode

(erfindungsgemäß)

Beispiel A

Wachstum bei Gras (Festuca pratensis)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen tropfnaß mit den Wirkstoffzubereitungen besprüht. Nach 2 Wochen wird bei allen Pflanzen der Zuwachs gemessen und in Prozent des Zuwachses der Kontrollpflanzen berechnet. Es bedeuten 100 % einen Zuwachs wie bei den Kontrollen, Werte unter 100 % geben eine Wuchshemmung, Werte über 100 % eine Wuchsförderung wieder.

Wirkstoffe, Wirkstoffkonzentrationen und Versuchsergebnisse gehen aus der folgenden Tabelle hervor.

Tabelle A

Wachstum bei Gras (Festuca pratensis)

| Wirkstoff | Konzentration in % | Zuwachs in % |
|---|---|---|
| - (Kontrolle) | - | = 100 |
| (A) | 0,05 | 68 *) |
| | 0,0125 | 88 |
| (B) | 0,05 | 88 |
| | 0,0125 | 93 |
| (I) | 0,05 | 29 *) |
| (erfindungsgemäß) | 0,0125 | 63 *) |

*) dunkelgrüne Blattfarbe

Beispiel B

Wachstum bei Gerste

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 2 Wochen wird bei allen Pflanzen der Zuwachs gemessen und in Prozent des Zuwachses der Kontrollpflanzen berechnet. Es bedeuten 100 % einen Zuwachs wie bei den Kontrollen, Werte unter 100 % geben eine Wuchshemmung, Werte über 100 % eine Wuchsförderung wieder.

Wirkstoffe, Wirkstoffkonzentrationen und Versuchsergebnisse gehen aus der folgenden Tabelle hervor.

Tabelle B

Wachstum bei Gerste

| Wirkstoff | Konzentration in % | Zuwachs in % |
|---|---|---|
| - | | |
| (Kontrolle) | - | = 100 |
| (A) | 0,05 | 68 *) |
| | 0,0125 | 76 |
| | 0,0031 | 84 |
| (B) | 0,05 | 89 |
| | 0,0125 | 89 |
| | 0,0031 | 93 |
| (I) | 0,05 | 51 **) |
| (erfindungsgemäß) | 0,0125 | 71 |
| | 0,0031 | 76 |

*) dunkgelgrüne Blattfarbe

**) Bestockung

Beispiel C

Wachstum bei Zuckerrüben

Lösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird bei allen Pflanzen der Zuwachs gemessen und in Prozent des Zuwachses der Kontrollpflanzen berechnet. Es bedeuten 100 % einen Zuwachs wie bei den Konrollen, Werte unter 100 % geben eine Wuchshemmung, Werte über 100 % eine Wuchsförderung wieder.

Wirkstoffe, Wirkstoffkonzentrationen und Versuchsergebnisse gehen aus der folgenden Tabelle hervor.

Tabelle C

Wachstum bei Zuckerrüben

| Wirkstoff | Konzentration in % | Zuwachs in % |
| --- | --- | --- |
| - | | |
| (Kontrolle) | - | = 100 |
| (B) | 0,05 | 45 |
| | 0,0125 | 75 |
| | 0,0031 | 104 |
| (I) | 0,05 | 0 |
| (erfindungsgemäß) | 0,0125 | 0 |
| | 0,0031 | 15 |

Beispiel D

Wachstum bei Sojabohnen

Lösungsmittel: 30 Gewichtsteile Dimethylformamid
Emulgator: 1 Gewichtsteil Polyoxyethylen-Sorbitan-Monolaurat

Zur Hestellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnen werden im Gewächshaus bis zur vollen Entfaltung des 1. Folgeblattes angezogen. In diesem Stadium werden die Pflanzen tropfnaß mit den Wirkstoffzubereitungen besprüht. Nach 2 Wochen wird bei allen Pflanzen der Zuwachs gemessen und in Prozent des Zuwachses der Kontrollpflanzen berechnet. Es bedeuten 100 % einen Zuwachs wie bei den Kontrollen, Werte unter 100 % geben eine Wuchshemmung, Werte über 100 % eine Wuchsförderung wieder.

Wirkstoffe, Wirkstoffkonzentrationen und Versuchsergebnisse gehen aus der folgenden Tabelle hervor.

Tabelle D

Wachstum bei Sojabohnen

| Wirkstoff | Konzentration in % | Zuwachs in % |
|---|---|---|
| - (Kontrolle) | - | = 100 |
| (B) | 0,05 | 17 *) |
| | 0,0125 | 66 *) |
| | 0,0031 | 66 *) |
| (I) (erfindungsgemäß) | 0,05 | 17 *) |
| | 0,0125 | 17 *) |
| | 0,0031 | 33 *) |

*) dunkelgrüne Blattfarbe

Beispiel E

Wachstum bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 2 Wochen wird bei allen Pflanzen der Zuwachs gemessen und in Prozent des Zuwachses der Kontrollpflanzen berechnet. Es bedeuten 100 % einen Zuwachs wie bei den Kontrollen, Werte unter 100 % geben eine Wuchshemmung, Werte über 100 % eine Wuchsförderung wieder.

Wirkstoffe, Wirkstoffkonzentrationen und Versuchsergebnisse gehen aus der folgenden Tabelle hervor.

Tabelle E

Wachstum bei Baumwolle

| Wirkstoff | Konzentration in % | Zuwachs in % |
|---|---|---|
| - | | |
| (Kontrolle) | - | = 100 |
| (B) | 0,05 | 39 *) |
| | 0,0031 | 78 *) |
| (I) | 0,05 | 13 *) |
| (erfindungsgemäß) | 0,0031 | 26 *) |

*) dunkelgrüne Blattfarbe

## Ansprüche

1) (-)-Antipode des (E)-1-(4-Chlorphenyl)-5-fluor-4,4-dimethyl-3-hydroxy-2-(1,2,4-triazol-1-yl)-pent-1-ens der Formel

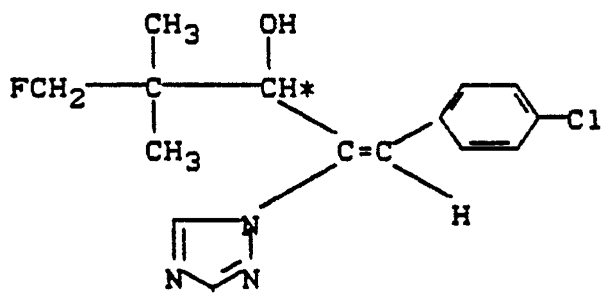

(I)

2. Verfahren zur Herstellung des (-)-Antipoden des (E)-1-(4-Chlorphenyl)-5-fluor-4,4-dimethyl-3-hydroxy-2-(1,2,4-triazol-1-yl)-pent-1-ens der Formel

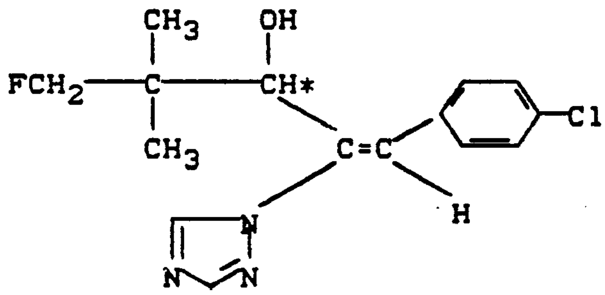

(I)

dadurch gekennzeichnet, daß man

a) in einer ersten Stufe racemisches (E)-1-(4-Chlorphenyl)-5-fluor-4,4-dimethyl-3-hydroxy-2-(1,2,4-triazol-1-yl)-pent-1-en der Formel

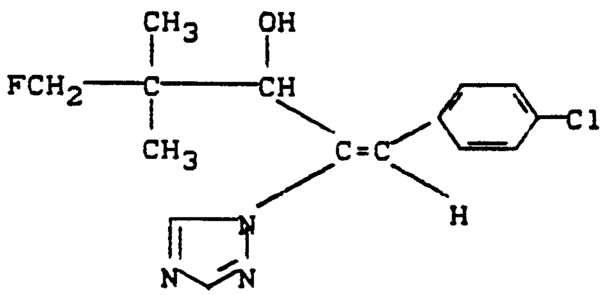

(Ia)

mit einem optisch aktiven Säurehalogenid der Formel

R -X -Hal (II)

in welcher

R für einen optisch aktiven Rest steht,

X für -CO-, $-SO_2$-oder $-O-CH_2-CO$-steht und

Hal für Halogen steht,

in Gegenwart eines Verdünnungsmittels und gegebenenfalls in Gegenwart von Basen umsetzt, dann die so erhaltenen diastereomeren Ester der Formel

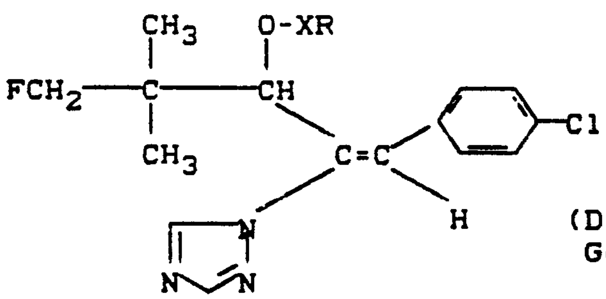

(III)
(Diastereomeren-Gemisch)

in welcher

R und X die oben angegebene Bedeutung haben, aufgrund ihrer unterschiedlichen physikalischen Eigenschaften trennt

und danach in einer zweiten Stufe den (-)-Antipoden des (E)-1-(4-Chlorphenyl)-5-fluor-4,4-dimethyl-3-hydroxy-2-(1,2,4-triazol-1-yl)-pent-1-ens aus dem entsprechenden Ester mit Hilfe von Basen in Gegenwart eines Verdünnungsmittels in Freiheit setzt,

oder

b) (E)-1-(4-Chlorphenyl)-5-fluor-4,4-dimethyl-2-(1,2,4-triazol-1-yl)-pent-1-en-3-on der Formel

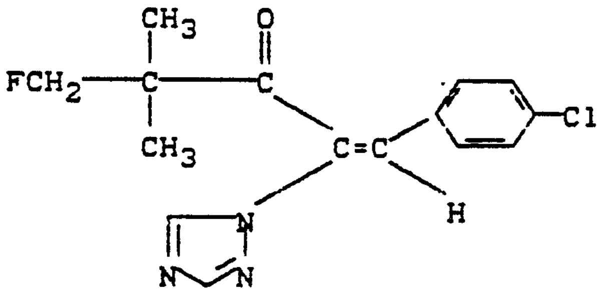

(IV)

mit einem komplexen Hydrid in Gegenwart eines inerten organischen Verdünnungsmittels sowie in Gegenwart eines chiralen Aminoalkohols und gegebenenfalls in Gegenwart eines Amins umsetzt.

3) Pflanzenwuchsregulierende Mittel, gekennzeichnet durch einen Gehalt an dem (-)-Antipoden des (E)-1-(4-Chlorphenyl)-5-fluor-4,4-dimethyl-3-hydroxy-2-(1,2,4-triazol-1-yl)-pent-1-ens der Formel (I).

4) Verwendung des (-)-Antipoden des (E)-1-(4-Chorphenyl)-5-fluor-4,4-dimethyl-3-hydroxy-2-(1,2,4-triazol-1-yl)-pent-1-ens der Formel (I) zur Regulierung des Pflanzenwachstums.

5) Verfahren zur Regulierung des Pflanzenwachstums, dadurch gekennzeichnet, daß man den (-)-Antipoden des (E)-1-(4-Chorphenyl)-5-fluor-4,4-dimethyl-3-hydroxy-2-(1,2,4-triazol-1-yl)-pent-1-ens der Formel (I) auf die Pflanzen und/oder deren Lebensraum ausbringt.

6) Verfahren zur Herstellung von pflanzenwuchsregulierenden Mitteln, dadurch gekennzeichnet, daß man den (-)-Antipoden des (E)-1-(4-Chlorphenyl)-5-fluor-4,4-dimethyl-3-hydroxy-2-(1,2,4-triazol-1-yl)-pent-1-ens der Formel (I) mit Streckmitteln und/oder oberflächenaktiven Stoffen vermischt.

Europäisches Patentamt

# EUROPÄISCHER RECHERCHENBERICHT

Nummer der Anmeldung

EP 86115196.7

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
| D,A | EP - A2 - 0 044 425 (BAYER)<br>* Ansprüche 1,7-10; Beispiele 12,13,28 *<br>---- | 1,3-6 | C 07 D 249/08<br>A 01 N 43/653 |
| | | | RECHERCHIERTE SACHGEBIETE (Int. Cl.4) |
| | | | C 07 D 249/00 |

Der vorliegende Recherchenbericht wurde für alle Patentansprüche erstellt.

| Recherchenort | Abschlußdatum der Recherche | Prüfer |
|---|---|---|
| WIEN | 10-02-1987 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument